# EUROPEAN PATENT APPLICATION

(11) **EP 4 510 651 A1**
(43) Date of publication of application: **19.02.2025**
(21) Application number: 23823037.9
(22) Date of filing: 09.06.2023
(51) Int. Cl.: H04W 4/50, A61B 5/00

(54) **SLEEP MONITORING METHOD AND DEVICE**

(30) Priority: 13.06.2022 CN 202210659490
(71) Applicant: Huawei Technologies Co., Ltd., Shenzhen, Guangdong 518129 (CN)
(72) Inventor: DONG, Wei, Shenzhen, Guangdong 518129 (CN); WANG, Qi, Shenzhen, Guangdong 518129 (CN); XIN, Jiapeng, Shenzhen, Guangdong 518129 (CN)
(74) Representative: Pfenning, Meinig & Partner mbB
(86) International application number: PCT/CN2023/099304
(87) International publication number: WO 2023/241469

(57) **Abstract**

A sleep monitoring method and a device are provided. The method includes: A routing device receives wireless signal flows from at least two smart devices in a space in which a target user is located, where the wireless signal flow includes channel state information for representing a sleep feature of the target user; the routing device determines a target device from the at least two smart devices based on the wireless signal flows of the at least two smart devices; and the routing device determines a sleep monitoring result of the target user based on a wireless signal flow of the target device. This helps improve accuracy of the sleep monitoring result.

## Description

### CROSS-REFERENCE TO RELATED APPLICATIONS

This application claims priority to Chinese Patent Application No. 202210659490.0, filed with the China National Intellectual Property Administration on June 13, 2022 and entitled "SLEEP MONITORING METHOD AND DEVICE", which is incorporated herein by reference in its entirety.

### TECHNICAL FIELD

This application relates to the field of electronic technologies, and in particular, to a sleep monitoring method and a device.

### BACKGROUND

Sleep is an important physiological process of a human body, and is a main way for the human body to eliminate physical fatigue. During deep sleep, tissues and organs of the body can be self-repaired through metabolism. People who have poor sleep quality or suffer from sleep disorders are prone to memory loss, lack of energy, and decreased immunity. In addition, many diseases in an incubation period and symptoms can be manifested in a sleep status, and the sleep status is also closely related to recovery of the diseases. Therefore, sleep monitoring and assessment have great application value in the medical field and the like.

Currently, in an existing contact-based sleep monitoring system, a monitoring device, for example, a wearable device or a smart pillow, needs to be additionally purchased. Such a contact-based device is likely to cause discomfort of a user. In addition, a sensor in the device is separated from a human body by an object such as a pillow or a sheet. Therefore, it is difficult to ensure accuracy of monitoring a sleep status of the user. In view of this, a non-contact-based sleep monitoring system is provided in the industry. However, the current sleep monitoring system does not consider various limiting factors of an actual scenario, and consequently accuracy of a sleep monitoring result is not high.

### SUMMARY

Embodiments of this application provide a sleep monitoring method and a device, to improve accuracy of a sleep monitoring result.

According to a first aspect, an embodiment of this application provides a sleep monitoring method. The method includes: A routing device receives wireless signal flows from at least two smart devices in a space in which a target user is located, where the wireless signal flow includes channel state information for representing a sleep feature of the target user; the routing device determines a target device from the at least two smart devices based on the wireless signal flows of the at least two smart devices; and the routing device determines a sleep monitoring result of the target user based on a wireless signal flow of the target device.

According to the foregoing solution, the routing device may first adaptively determine, from the at least two smart devices based on the wireless signal flows of the at least two smart devices, a target device suitable for sleep monitoring, and then determine the sleep monitoring result of the target user based on a wireless signal flow of the target device. This helps improve accuracy of the sleep monitoring result.

In a possible design, the routing device may determine the target device from the at least two smart devices in a plurality of possible implementations:
Manner 1: The routing device may determine, from the at least two smart devices as target devices, top N smart devices whose respiration detection rates are greater than or equal to a first threshold and whose respiration detection rates are highest, where N is a positive integer. A larger respiration detection rate of a smart device indicates that the smart device is more sensitive to a body motion status of the user during sleep. In Manner 1, a target device suitable for sensing the body motion status of the user during sleep may be selected.
Manner 2: The routing device may determine N smart devices closest to the target user from the at least two smart devices as target devices. A smart device closer to the target user is more sensitive to a body motion status of the user during sleep. In Manner 2, a target device suitable for sensing the body motion status of the user during sleep may be selected.
Manner 3: The routing device may randomly select N smart devices from the at least two smart devices as target devices.

In a possible design, the wireless signal flow of the target device carries Wi-Fi CSI information. That the routing device determines a sleep monitoring result of the target user based on a wireless signal flow of the target device includes: The routing device determines body motion feature information and respiration feature information of the target user based on the Wi-Fi CSI information; and determines the sleep monitoring result of the target user based on the body motion feature information and the respiration feature information, where the sleep monitoring result includes each sleep stage of the user and a sleep quality assessment result.

In a possible design, the method further includes: If a respiration detection rate of the target device is less than the first threshold within preset duration, the routing device sends first prompt information to a terminal device, where the first prompt information is used to prompt the user to adjust the target device. If the respiration detection rate of the target device is less than the first threshold, it indicates that the target device is insensitive to the body motion status of the user during sleep. The routing device may remind, by sending the first prompt information to the terminal device, the user in time to adjust the target device.

In a possible design, the method further includes: The routing device monitors a respiration detection rate of another smart device other than the target device in the at least two smart devices; and if a smart device whose respiration detection rate is greater than the first threshold exists in the another smart device, the routing device sends second prompt information to the terminal device, where the second prompt information is used to prompt the user to replace the target device. According to this design, the user can be reminded to replace the target device with a target device that is more sensitive to the body motion status of the user during sleep, so that accuracy of the sleep monitoring result can be improved.

In a possible design, the method further includes: The routing device sends the sleep monitoring result of the target user to the terminal device. In this way, the user can view the sleep monitoring result on the terminal device side.

In a possible design, the space in which the target user is located further includes at least one other user. That the routing device determines a sleep monitoring result of the target user based on a wireless signal flow of the target device includes: The routing device separates a respiration signal of the target user and a respiration signal of the at least one other user from the wireless signal flow; the routing device determines the sleep monitoring result of the target user based on the respiration signal of the target user; and the routing device determines a sleep monitoring result of each of the at least one other user based on a respiration signal of each of the at least one other user. In this way, an implementation of sleep monitoring in a multi-person scenario is provided, and accuracy of a monitoring result can also be ensured.

In a possible design, the method further includes: The routing device sends a room-level sleep monitoring result to the terminal device, where the room-level sleep monitoring result includes a room-level sleep state, room-level sleep duration, and fall-asleep time points, respiration rates, and respiration stability of the target user and each user in the at least one other user.

According to a second aspect, an embodiment of this application further provides an electronic device, including a processor, a memory, and one or more programs. The one or more programs are stored in the memory. The one or more programs include instructions. When the instructions are executed by the processor, the electronic device is enabled to perform steps of the method provided in the first aspect or any one of the designs in the first aspect.

According to a third aspect, an embodiment of this application further provides an apparatus. The apparatus includes modules/units that perform the method in any possible design in any one of the foregoing aspects. These modules/units may be implemented by hardware, or may be implemented by hardware by executing corresponding software.

According to a fourth aspect, an embodiment of this application further provides a computer-readable storage medium. The computer-readable storage medium includes a computer program. When the computer program is run on a terminal, the terminal is enabled to perform the method in any possible design in any one of the foregoing aspects.

According to a fifth aspect, an embodiment of this application further provides a computer program product. When the computer program product is run on a terminal, the terminal is enabled to perform the method in any possible design in any one of the foregoing aspects.

These or other aspects of this application are more concise and easier to understand in descriptions of the following embodiments.

### BRIEF DESCRIPTION OF DRAWINGS

FIG. 1 is a diagram of a scenario to which embodiments of this application are applied;
FIG. 2 is a diagram of a communication system according to an embodiment of this application;
FIG. 3 is a diagram of a hardware structure of a routing device according to an embodiment of this application;
FIG. 4 is a schematic flowchart of a sleep monitoring method according to an embodiment of this application;
FIG. 5 is a diagram of setting a short-time detection mode according to an embodiment of this application;
FIG. 6 is a schematic flowchart of determining a sleep monitoring result according to an embodiment of this application;
FIG. 7 is a diagram of aggregation degrees of a target user in a static state and a motion state according to an embodiment of this application;
FIG. 8 is a diagram of a respiration rate according to an embodiment of this application;
FIG. 9 is a schematic flowchart of a sleep monitoring method according to an embodiment of this application;
FIG. 10 is a diagram of respiration signals of a plurality of users according to an embodiment of this application; and
FIG. 11 is a diagram of a hardware structure of a routing device according to an embodiment of this application.

### DESCRIPTION OF EMBODIMENTS

Currently, in an existing contact-based sleep monitoring system, a monitoring device, for example, a wearable device or a smart pillow, needs to be additionally purchased. Such a contact-based device is likely to cause discomfort of a user. In addition, a sensor in the device is separated from a human body by an object such as a pillow or a sheet. Therefore, it is difficult to ensure accuracy of monitoring a sleep status of the user. However, a non-contact-based sleep monitoring system provided in the industry does not consider various limiting factors of an actual scenario, and consequently perception precision of sleep monitoring is not high.

In view of this, an embodiment of this application provides a sleep monitoring method. The method is applied to a routing device. At least two smart devices exist in a space in which a target user is located. The routing device may receive at least two wireless signal flows from the at least two smart devices, where the wireless signal flow includes channel state information for representing a sleep feature of the target user. The routing device may determine at least one target device from the at least two smart devices based on the at least two wireless signal flows, and then determine a sleep monitoring result of the target user based on a wireless signal flow of the at least one target device.

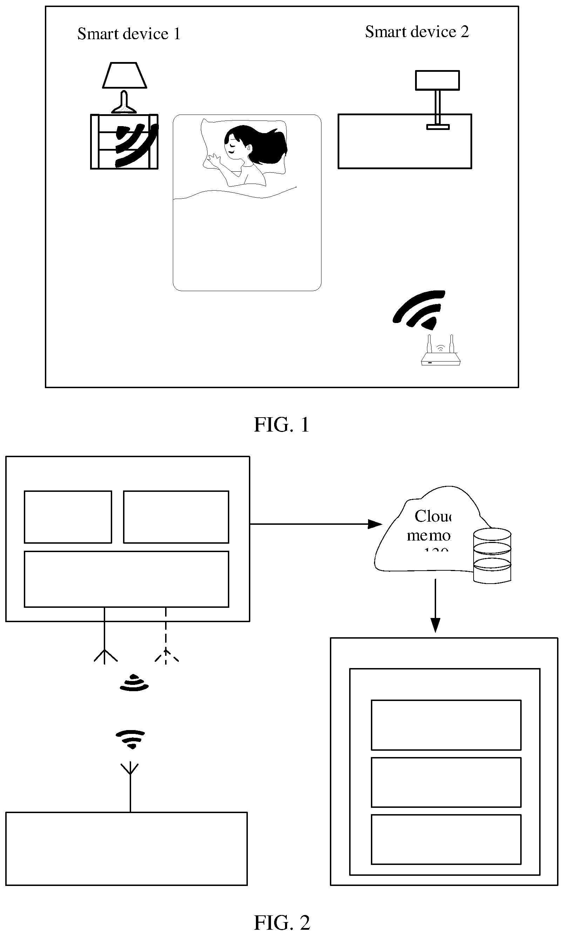

The technical solutions in embodiments of this application may be applied to a scenario shown in FIG. 1. A routing device and at least two smart devices are deployed in a space in which a target user is located. Two smart devices shown in FIG. 1 as examples are a smart device 1 and a smart device 2. The smart device 1 and the smart device 2 each may send a wireless signal. Propagation of the wireless signal in the space is affected by an action, respiration, and the like of the target user. The routing device may adaptively select a smart device as a target device, for example, select the smart device 1 as the target device. Then, the routing device may perform sleep monitoring on the target user by collecting the wireless signal sent by the smart device 1. In this way, accuracy of a sleep monitoring result can be improved.

It should be understood that, in embodiments of this application, the at least two smart devices and the target user are located in a same space, for example, in a same room. The routing device and the target user may be located in a same space, or may be located in different spaces. For example, the target user, the routing device, and a plurality of smart devices are all located in a bedroom. For another example, the target user and a plurality of smart devices are located in a bedroom, and the routing device is located in a living room. The target user, the routing device, and the plurality of smart devices are deployed in one space, so that precision of the sleep monitoring result can be higher.

The following provides a communication system to which embodiments of this application are applied. The communication system includes a routing device and at least two smart devices. The routing device and the at least two smart devices may perform local area network (wireless local area network, WLAN) communication based on a wireless fidelity (wireless fidelity, Wi-Fi) connection, and may be wirelessly connected. FIG. 2 shows examples of a routing device 110 and a smart device 120. Optionally, the communication system may further include a cloud memory 130 and a terminal device 140.

The routing device 110 may be a router, or may be another terminal or an electronic device having a Wi-Fi access function. FIG. 2 is a diagram of a hardware structure of the routing device. On the basis of FIG. 2, the routing device may alternatively exist in another variant structure manner.

As shown in FIG. 2, the routing device 110 may include a processor 111, an internal memory 112, and a wireless communication module 113. It may be understood that the structure shown in embodiments of this application does not constitute a specific limitation on the routing device 110. In some other embodiments of this application, the routing device 110 may include more or fewer components than those shown in FIG. 2, or some components may be combined, or some components may be split, or there may be a different component layout. The components shown in FIG. 2 may be implemented by hardware, software, or a combination of software and hardware.

The following describes in detail the components of the routing device 110 shown in FIG. 2.

The processor 111 may include one or more processing units. For example, the processor 111 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, a neural-network processing unit (neural-network processing unit, NPU), and/or the like. Different processing units may be independent components, or may be integrated into one or more processors. The controller may be a nerve center and a command center of the routing device 110. The controller may generate an operation control signal based on an instruction operation code and a time sequence signal, to implement control on instruction fetching and execution.

A memory may be further disposed in the processor 111, and is configured to store instructions and data. In some embodiments, the memory in the processor 111 is a cache. The memory may store instructions or data that has been used or cyclically used by the processor 111. If the processor 111 needs to use the instructions or the data again, the processor 111 may directly invoke the instructions or the data from the memory, so that repeated access can be avoided, and a waiting time of the processor 111 can be reduced, thereby improving system efficiency.

In embodiments of this application, an example in which the routing device 110 is the routing device in the scenario shown in FIG. 1 is used. The processor 111 in the routing device 110 may determine, based on at least two received wireless signal flows from at least two smart devices, a target device for performing sleep monitoring on a target user, and then the processor 111 determines a sleep feature of the target user based on a wireless signal flow from the target device, where the sleep feature is, for example, a body motion feature, a respiration feature, and the like that are described below. Then, the processor 111 obtains a sleep monitoring result through calculation based on the sleep feature.

Optionally, the processor 111 may further control the wireless communication module 113 to store the sleep monitoring result into the cloud memory.

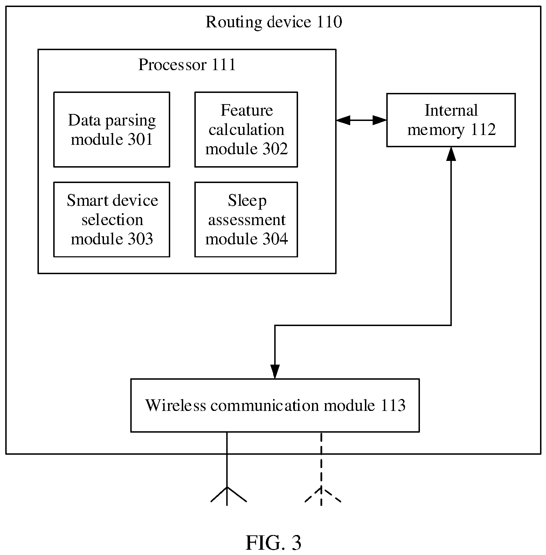

Based on the diagram of the structure of the routing device 110 shown in FIG. 2, as shown in FIG. 3, the processor 111 may include a plurality of functional modules: a data parsing module 301, a feature calculation module 302, a smart device selection module 303, and a sleep assessment module 304. Functions of the modules are as follows:
The data parsing module 301 is configured to: read at least two wireless signal flows in the internal memory 112, then parse the at least two wireless signal flows to obtain Wi-Fi CSI information in the at least two wireless signal flows, and send the Wi-Fi CSI information to the feature extraction module.

The feature calculation module 302 is configured to: read the Wi-Fi CSI information, calculate a body motion feature and a respiration feature based on the Wi-Fi CSI information, and store the calculated features into the internal memory 112.

The smart device selection module 303 is configured to: read the body motion feature and the respiration feature from the internal memory 112, and output a smart device suitable for sleep monitoring.

The sleep assessment module 304 is configured to: read the body motion feature and the respiration feature from the internal memory 112, calculate a sleep monitoring result based on the features, and calculate respiration rate distribution of the whole night, where the sleep monitoring result includes a fall-asleep time point, a get-up time point, and sleep stage classification. Then, the sleep assessment module 304 stores the sleep monitoring result into the internal memory 112.

The internal memory 112 may be configured to store computer-executable program code. The executable program code includes instructions. The processor 111 runs or executes the instructions stored in the internal memory 112, to perform various function applications of the routing device 110 and data processing. The internal memory 112 may include a program storage area and a data storage area. The program storage area may store program instructions. The data storage area may store data (for example, the sleep feature, the sleep monitoring result, and the like) created in a process of using the routing device 100. In addition, the internal memory 112 may include a high-speed random access memory, or may include a nonvolatile memory, for example, a magnetic disk storage device, a flash memory, another nonvolatile solid-state storage device, or the like.

For example, the internal memory 112 may be further configured to store program code of the sleep monitoring method provided in embodiments of this application. When the processor 111 accesses and runs the program code of the sleep monitoring method, it may be implemented that the routing device 110 determines, based on the received wireless signal flows of the at least two smart devices 120, the target device for performing sleep monitoring on the target user, and then obtains the sleep feature and the sleep monitoring result based on the wireless signal flow from the target device.

The wireless communication module 113 may provide a wireless communication solution that is applied to the routing device 110 and that includes a wireless local area network (for example, a Wi-Fi network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 113 may be one or more components integrating at least one communication processing module. The wireless communication module 113 receives an electromagnetic wave through an antenna, performs frequency modulation and filtering processing on the electromagnetic wave signal, and sends a processed signal to the processor 111. The wireless communication module 113 may further receive a to-be-sent signal from the processor 111, perform frequency modulation and amplification on the signal, and convert the signal into an electromagnetic wave for radiation through the antenna.

For example, in embodiments of this application, the wireless communication module 113 may obtain the at least two wireless signal flows sent by the at least two smart devices, and store the at least two wireless signal flows into the internal memory 112. The processor 111 may obtain the at least two wireless signal flows from the internal memory 112.

Although not shown in FIG. 1, the routing device 110 may further include a peripheral interface, configured to provide various interfaces for external input/output devices. The routing device 110 may further include a charging management module that supplies power to each component. Optionally, the routing device 110 may further include an indicator, for example, an indicator configured to indicate that a power supply is connected, or an indicator configured to indicate signal strength. Details are not described herein.

All the following embodiments may be implemented in the routing device 110 having the foregoing structure.

The smart device 120 may send a wireless signal flow, so that the routing device may obtain a sleep monitoring result based on the received wireless signal flow. Propagation of the wireless signal flow in a space is affected by an action, respiration, and the like of the target user. The smart device 120 may be a terminal device (terminal equipment), user equipment (user equipment, UE), a mobile station (mobile station, MS), a mobile terminal (mobile terminal, MT), another portable device, or the like.

The cloud memory 130 may store the sleep monitoring result sent by the routing device.

An application (application, APP) may be deployed on the terminal device 140. As shown in FIG. 2, the app may include a plurality of modules: a configuration module 141, a result obtaining module 142, and an interface rendering module 143. Functions of the modules are as follows:
The configuration module 141 is configured to configure a time at which a sleep monitoring system starts to run and a time at which the sleep monitoring system ends running, and is configured to confirm entering or exiting a short-time detection mode.

The result obtaining module 142 is configured to obtain a sleep assessment result from the cloud memory 130.

The interface rendering module 143 is configured to present a sleep result of the whole night on the APP.

For example, the terminal device 140 may be a mobile phone (mobile phone), a tablet computer (pad), a computer with a wireless transceiver function, a virtual reality (virtual reality, VR) terminal, an augmented reality (augmented reality, AR) terminal, a wireless terminal in industrial control (industrial control), a wireless terminal in self-driving (self-driving), a wireless terminal in remote surgery (remote medical surgery), a wireless terminal in a smart grid (smart grid), a wireless terminal in transportation safety (transportation safety), a wireless terminal in a smart city (smart city), a wireless terminal in a smart home (smart home), or the like. Specific technologies and specific device forms that are used by the smart device and the terminal device are not limited in this application. For example, the terminal device in embodiments of this application includes but is not limited to a device running HarmonyOS^{®}, iOS^{®}, Android^{®}, Microsoft^{®}, or another operating system.

It should be understood that the terms "system" and "network" may be used interchangeably in embodiments of this application. "At least one" means one or more, and "a plurality of" means two or more. "And/or" describes an association relationship between associated objects, and indicates that three relationships may exist. For example, A and/or B may indicate the following cases: Only A exists, both A and B exist, and only B exists, where A and B may be singular or plural. The character "/" usually indicates an "or" relationship between the associated objects. "At least one of the following items (pieces)" or a similar expression thereof refers to any combination of these items, including a single item (piece) or any combination of a plurality of items (pieces). For example, at least one item (piece) of a, b, or c may indicate a, b, c, a and b, a and c, b and c, or a, b, and c.

In addition, unless otherwise specified, "first" and "second" in embodiments of this application are merely intended for a purpose of description, and shall not be understood as an indication or implication of relative importance or an implicit indication of a quantity of indicated technical features. Therefore, a feature defined with "first" or "second" may explicitly or implicitly include one or more features.

With reference to the foregoing embodiments and accompanying drawings, an embodiment of this application provides a sleep monitoring method, and the method may be implemented in any routing device 110 shown in FIG. 1 to FIG. 3.

### Embodiment 1

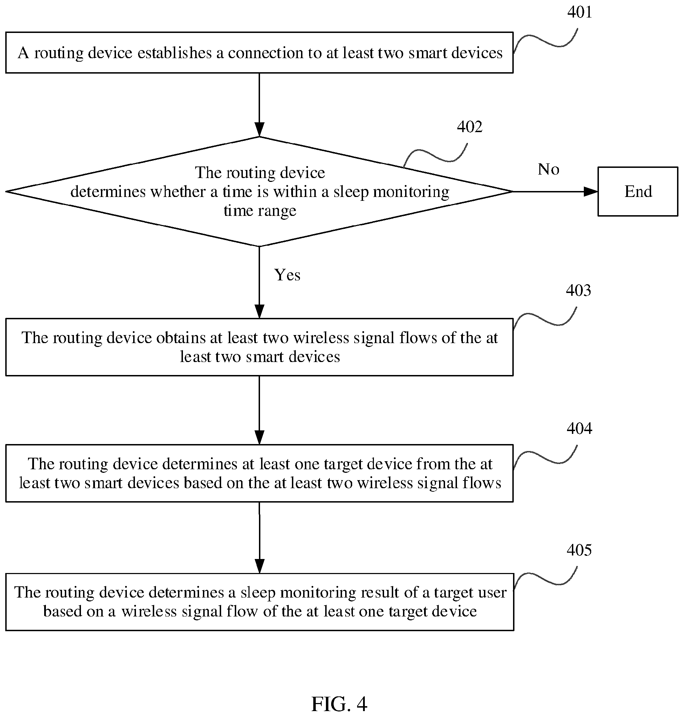

FIG. 4 is a schematic flowchart of a sleep monitoring method according to this application. A process of the method may include the following steps.

Step 401: A routing device establishes a connection to at least two smart devices.

Step 402: The routing device determines whether a time is within a sleep monitoring time range.

For example, a sleep monitoring system is deployed on the routing device. A user may configure a time at which the sleep monitoring system starts to run and a time at which the sleep monitoring system ends running, where a time interval between the time at which the sleep monitoring system starts to run and the time at which the sleep monitoring system ends running is the sleep monitoring time range. For example, the user may configure the time at which the sleep monitoring system starts to run to 7 o'clock every night and the time at which the sleep monitoring system ends running to 9 o'clock every morning. A specific value of the time of starting to run and/or a specific value of the time of ending running may be a default value of the sleep monitoring system on the routing device, or may be manually configured by the user. This is not limited in embodiments of this application.

Step 403: If the time is within the sleep monitoring time range, the routing device obtains at least two wireless signal flows of the at least two smart devices, or if the time is not within the sleep monitoring time range, ends the process.

In embodiments of this application, the at least two smart devices are located in a space in which a target user is located, each smart device in the at least two smart devices corresponds to one wireless signal flow, and the wireless signal flow of each smart device includes channel state information for representing a sleep feature of the target user.

Step 404: The routing device determines at least one target device from the at least two smart devices based on the at least two wireless signal flows.

There may be a plurality of possible implementations of determining the at least one target device from the at least two smart devices in step 404.

In a possible implementation a1, if the sleep monitoring system enters a short-time detection mode, the routing device may determine the at least one target device based on short-time detection results of the at least two smart devices.

Optionally, before step 404, the method may further include a step: The routing device determines whether the sleep monitoring system enters the short-time detection mode.

A specific implementation in which the sleep monitoring system enters the short-time detection mode includes but is not limited to any one of the following:
In a feasible implementation, the sleep monitoring system may automatically enter the short-time detection mode or exit the short-time detection mode. For example, it may be configured that the sleep monitoring system enters the short-time detection mode when the sleep monitoring system starts to run, or may be configured that the sleep monitoring system enters the short-time detection mode within first preset duration after the monitoring system starts to run; and the sleep monitoring system exits the short-time detection mode after second preset duration after entering the short-time detection mode. For example, the first preset duration is set to 60s, and the second preset duration is set to 5 min. Specific values of the first preset duration and the second preset duration may be set based on an actual requirement. This is not limited herein. It should be understood that the short-time detection mode may alternatively be turned on when the sleep monitoring system on the router is configured for the first time. In other words, the target device may be first determined during the first configuration, and the target device may be adjusted at any time when sleep monitoring is subsequently performed on the target user.

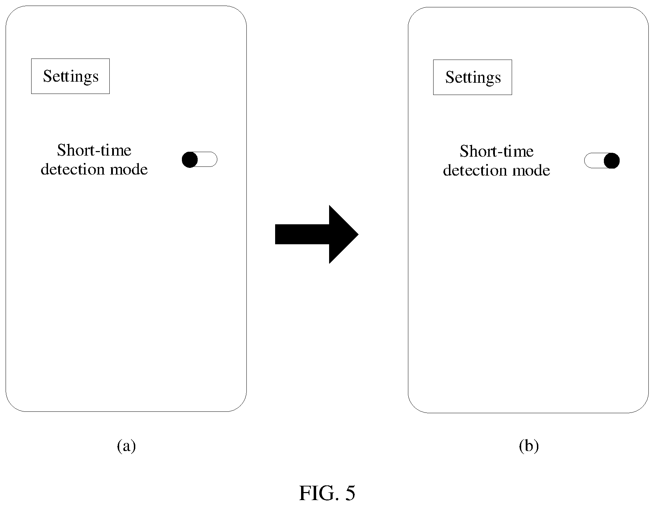

In another feasible implementation, the user may manually select to enter the short-time detection mode. For example, the routing device is connected to a terminal device, the user may control the routing device by using an application on the terminal device, and a control used to turn on or turn off the short-time detection mode is displayed on a display of the terminal device. As shown in (a) in FIG. 5, the control is off. When the terminal device detects an operation instruction for the control, as shown in (b) in FIG. 5, the short-time detection mode is turned on. The routing device starts to perform short-time detection to select, from the at least two smart devices, a target device used to detect a sleep status of the target user. When the short-time detection mode needs to be exited, for example, after a short-time detection result is obtained, the user manually turns off the control. Alternatively, a switch of the short-time detection mode may be provided on the routing device, and the user may manually turn on the switch, so that the sleep monitoring system enters the short-time detection mode. When the short-time detection mode needs to be exited, the switch is manually turned off.

During short-time detection, the user can lie still on the bed and keep breathing normally. After the sleep monitoring system enters the short-time detection mode, the routing device may obtain wireless signal flows from the at least two smart devices, for example, obtain wireless signal flows from the at least two smart devices within duration of 1 min; and then the routing device determines a respiration detection rate of each smart device based on the obtained wireless signal flow.

A higher respiration detection rate of a smart device indicates that a wireless signal is more sensitive to a body motion (including a rise and fall of the thoracic cavity), and therefore it indicates that the wireless signal of the smart device is more suitable for sensing a body motion status of the user during sleep. For example, the routing device may determine, as target devices, top N smart devices whose respiration detection rates are greater than or equal to a first threshold and whose respiration detection rates are highest, where N is a positive integer. In this way, the routing device can select a target device suitable for sensing the body motion status of the user during sleep. This can improve accuracy of a sleep monitoring result.

In some other embodiments, if the routing device does not enter the short-time detection mode, the routing device may determine the target device in a possible implementation a2 or a possible implementation a3.

In the possible implementation a2, the routing device may determine the at least one target device based on a distance between a location of user equipment and a location of each of the at least two smart devices.

When the target user sleeps, a smart device closer to the user is usually more sensitive to a body motion of the target user. The smart device closer to the user equipment may be preferentially selected as the target device. For example, N smart devices closest to the target user are determined as target devices. In this way, a target device suitable for sensing the body motion of the user during sleep is selected. This can improve accuracy of a sleep monitoring result.

In the possible implementation a3, alternatively, the routing device may randomly select N smart devices as target devices, and then adjust the target devices when subsequently monitoring a sleep status of the target user. For example, a respiration detection rate of a smart device is determined based on a wireless signal flow collected in a monitoring process, then a target device is re-determined based on the respiration detection rate, and then step 405 is performed based on a wireless signal flow of the re-determined target device.

Step 405: The routing device determines a sleep monitoring result of the target user based on a wireless signal flow of the at least one target device.

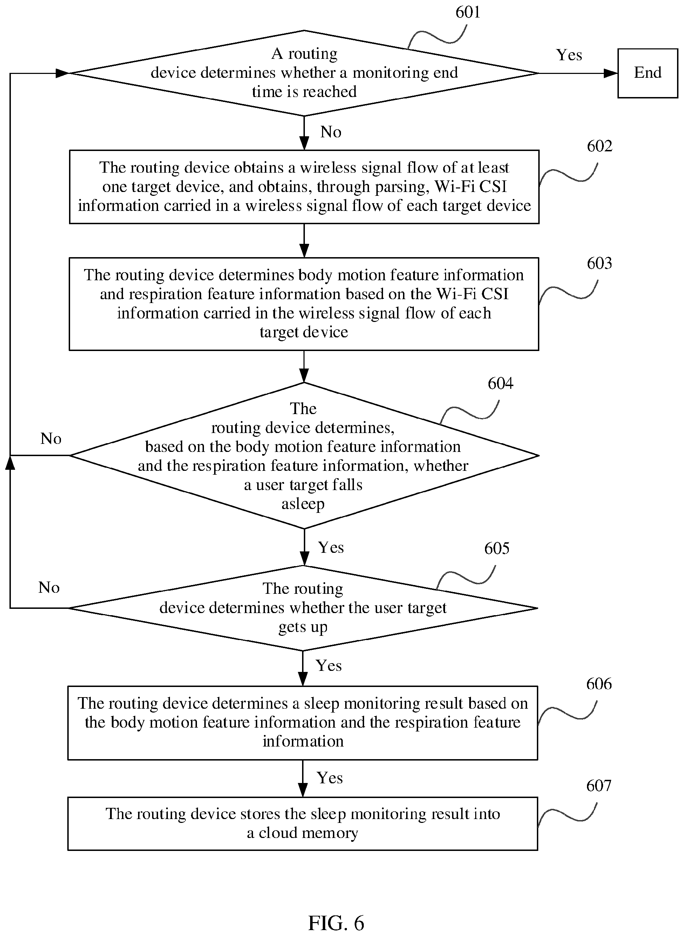

FIG. 6 shows a detailed process of step 405. Specifically, the following steps are included.

Step 601: The routing device determines whether a monitoring end time is reached, and if yes, ends the monitoring process ends, or if no, performs step 602.

Step 602: The routing device obtains the wireless signal flow of the at least one target device, and obtains, through parsing, Wi-Fi channel state information (channel state information, CSI) carried in a wireless signal flow of each target device.

Step 603: The routing device determines body motion feature information and respiration feature information based on the Wi-Fi CSI information carried in the wireless signal flow of each target device.

For example, the body motion feature information may include a body motion rate.

Body motion affects an aggregation degree of a CSI signal, and an indicator for constructing the aggregation degree is, for example, a variance of N consecutive CSI phase differences.

For example, the routing device includes two antennas, and a wireless signal flow received within 1s includes 50 packets. The routing device may obtain a phase 1 based on a packet 1 received through an antenna 1, and may obtain a phase 2 based on a packet 2 received through an antenna 2. Therefore, the packet 1 corresponds to a phase difference, that is, a difference between the phase 1 and the phase 2. Further, it is determined that the wireless signal flow received within 1s corresponds to 50 phase differences. The body motion status of the target user may be determined based on an aggregation degree of the 50 phase differences.

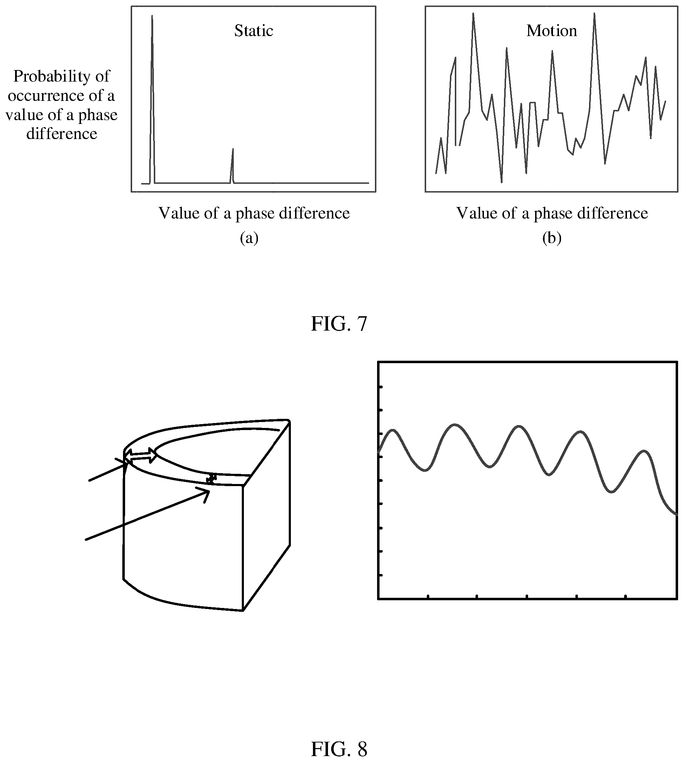

FIG. 7 is a diagram of aggregation degrees of the target user in a static state and a motion state. A horizontal coordinate represents a value of a phase difference, and a vertical coordinate represents a probability of occurrence of the value of the phase difference.

As shown in (a) in FIG. 7, values of phase differences are concentrated, indicating that the target user is in the static state. As shown in (b) in FIG. 7, values of phase differences are not concentrated but scattered, indicating that the target user is in the motion state.

Then, the routing device may determine a body motion rate based on a body motion status obtained through monitoring within t minutes. The body motion rate indicates a quantity of body motion times per second within the t minutes. For example, if M body motion times occur within the t minutes, the body motion rate may be represented as M/(60*t).

The following describes a specific implementation of determining the respiration feature information.

For example, the respiration feature information may include a respiration rate, a respiration detection rate, respiration stability, and the like. The respiration rate indicates a quantity of breaths per minute. The respiration detection rate indicates a ratio of a quantity of times of successful detection to a quantity of times of actual detection. The respiration stability indicates a variance of respiration rates, and may be used to measure a respiration fluctuation feature.

Because a cyclical rise and fall of the body of the target user during respiration causes a cyclical change of a wireless signal, the respiration rate may be obtained according to a method for capturing a signal cycle, such as FFT.

(a) in FIG. 8 is a diagram of simulating rising and falling of a thoracic cavity of the target user during respiration. When the target user exhales, the thoracic cavity expands outward, so that a crest shown in (b) in FIG. 8 appears in a wireless signal. When the target user inhales, the thoracic cavity contracts inward, so that a trough of the wireless signal shown in (b) in FIG. 8 appears in the wireless signal. According to a diagram of a wireless signal change shown in (b) in FIG. 8, the target user takes five breathes within 30 seconds. In this case, an obtained respiration rate is 10 breaths/min.

The routing device may determine respiration stability based on a respiration rate within a period of time. For example, a respiration rate of the target user in each 1 min within 20 minutes is counted. For example, the target user takes 12 breathes in a 1st 1 min, takes 20 breathes in a 2nd 1 min, and takes 15 breathes in a 3rd 1 min, until a quantity of breaths in a 20th 1 min is counted. In this case, a variance of the respiration rates within the 20 min may be determined. If the variance is large, it indicates that the respiration is unstable. If the variance is small, it indicates that the respiration is unstable.

Step 604: The routing device determines, based on the body motion feature information and the respiration feature information, whether the user target falls asleep, and if yes, performs step 605, or if no, performs step 601.

In a possible implementation, the routing device determines, based on the body motion feature information and the respiration feature information, a specific work and rest stage in which the target user is located.

For example, the target user has minimal body motions and a high respiration detection rate in a fall-asleep stage. When determining that the body motion rate is less than a second threshold and the respiration detection rate is greater than a third threshold, the routing device determines that the target user is in the fall-asleep stage.

The target user has infrequent body motions or a low respiration detection rate in a nocturnal wakefulness stage. When determining that the body motion rate is greater than the first threshold and less than a fourth threshold, and the respiration detection rate is less than a third threshold, the routing device determines that the target user is in the nocturnal wakefulness stage.

After the target user gets up, the target user has frequent body motions and a low respiration detection rate. When determining that the body motion rate is greater than a fourth threshold and the respiration detection rate is less than a third threshold, the routing device determines that the target user is in a get-up stage.

Step 605: The routing device determines whether the user target gets up, and if yes, performs step 606, or if no, performs step 601.

Step 606: The routing device determines the sleep monitoring result based on the body motion feature information and the respiration feature information.

For example, the sleep monitoring result may include each stage of the target user and a sleep quality assessment result.

The sleep quality assessment result may present respiration rate distribution of the target user in the whole night. Whether respiration is stable in the whole night, that is, respiration stability, and whether respiration is abnormal (for example, sleep apnea, or the like) are determined based on the respiration rate distribution.

Step 607: The routing device stores the sleep monitoring result into a cloud memory.

In some other embodiments, after the at least one target device is determined in step 404, a wireless signal flow of another smart device may be further monitored. In this way, when the target device needs to be adjusted, the target device is replaced with a smart device that is more suitable for sleep monitoring. Optionally, when it is determined that a respiration detection rate of the target device within T1 hours is less than the first threshold, it may be determined that the target device needs to be adjusted.

For example, the at least two smart devices are four smart devices: a smart device 1, a smart device 2, a smart device 3, and a smart device 4. After the routing device determines that the smart device 1 and the smart device 2 are target devices, for example, when a respiration detection rate of the smart device 1 is less than the first threshold within the T1 hours, the routing device may output first prompt information to the terminal device, where the first prompt information is used to prompt the user to adjust the target device. After the user views the first prompt information, the user may adjust a location of the target device, for example, adjust the location of the target device to a location closer to the bed of the target user, to increase the respiration detection rate corresponding to the target device. The routing device may further monitor wireless signal flows of the smart device 3 and the smart device 4 for T2 minutes. If a respiration detection rate of the smart device 3 or the smart device 4 is greater than the first threshold, the smart device 3 or the smart device 4 replaces the smart device 1 as the target device, or the terminal device prompts the user whether to replace the target device. In this case, the user may choose whether to replace the target device.

In the foregoing embodiment, an example in which the routing device is connected to at least two smart devices is used for description. In some other embodiments, when there is only one smart device in the space in which the target user is located, after the routing device establishes a connection to the smart device, the routing device may perform sleep monitoring on the target user based on a wireless signal flow of the smart device. If the routing device determines that a respiration detection rate corresponding to the smart device is less than the first threshold, prompt information may be output by using the terminal device, where the prompt information is used to prompt the user that the smart device is not suitable for sleep monitoring, or prompt the user to adjust a location of the smart device, for example, adjust the location of the smart device to a location closer to the bed of the target user.

### Embodiment 2

A difference between Embodiment 2 and Embodiment 1 in the foregoing lies in that, in Embodiment 2, a case in which a plurality of persons are in one room is considered. In this case, when a routing device may separate a respiration signal of each person from a wireless signal flow received from a smart device, and then perform sleep monitoring for the respiration signal of each person.

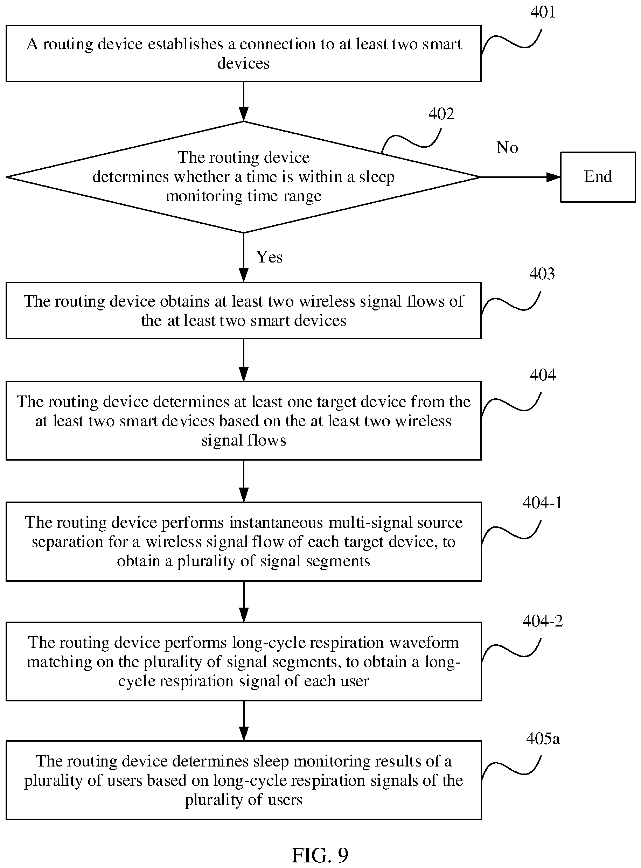

FIG. 9 is a schematic flowchart of a sleep monitoring method according to this application. A difference between FIG. 9 and FIG. 4 in Embodiment 1 in the foregoing lies in that: Step 404-1 and step 404-2 are added between step 404 and step 405, and step 405 is replaced with step 405a. To save space, the following describes only step 404-1, step 404-2, and step 405a in FIG. 9. For other steps in FIG. 9, refer to the description in FIG. 4.

Step 404-1: The routing device performs instantaneous multi-signal source separation for a wireless signal flow of each target device, to obtain a plurality of signal segments.

Herein, a signal processing algorithm, for example, an independent component analysis (independent component correlation algorithm, ICA) algorithm, may be considered to be used in an instantaneous multi-signal source separation process. Respiration signals of a plurality of persons are separated every S seconds, so that a respiration signal of each person is extracted from a CSI signal, to obtain a plurality of S-second signal segments. Then, the respiration signal of each person is extracted from each S-second signal segment.

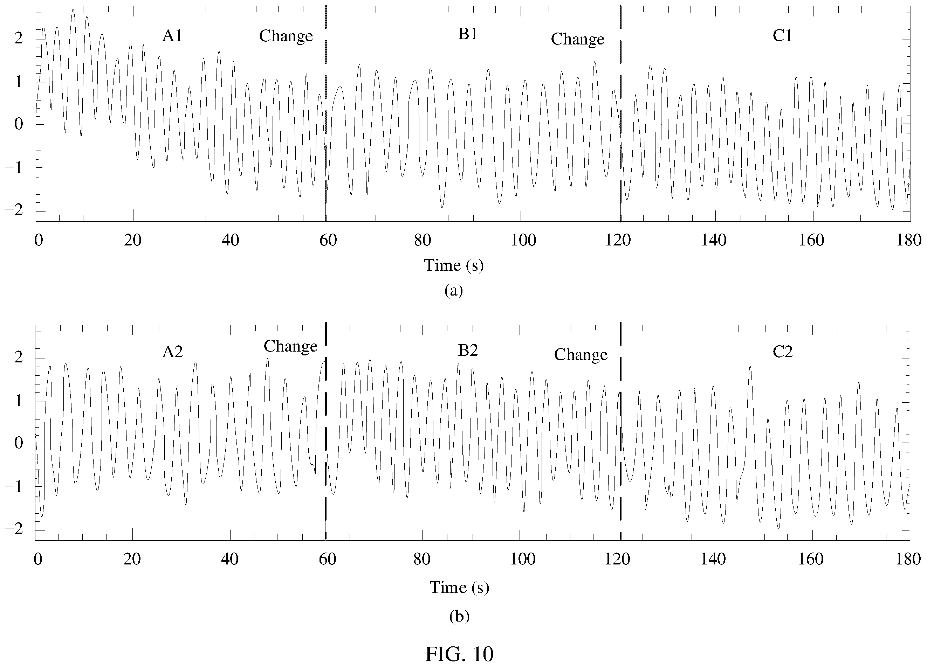

For example, S is 60 seconds. For example, a 3-min signal is obtained, and the signal includes respiration signals of a user 1 and a user 2. A respiration signal A1 shown in (a) in FIG. 10 and a respiration signal A2 shown in (b) in FIG. 10 may be extracted from a 1st 60-second signal segment A included in the 3-min signal, a respiration signal B1 shown in (a) in FIG. 10 and a respiration signal B2 shown in (b) in FIG. 10 may be extracted from a 2nd 60-second signal segment B included in the 3-min signal, and a respiration signal C1 shown in (a) in FIG. 10 and a respiration signal C2 shown in (b) in FIG. 10 may be extracted from a 3rd 60-second signal segment C included in the 3-min signal.

Step 404-2: The routing device performs long-cycle respiration waveform matching on the plurality of signal segments, to obtain a long-cycle respiration signal of each user.

The foregoing example is still used. Waveform matching is performed on each of the respiration signal A1, the respiration signal A2, the respiration signal B1, the respiration signal B2, the respiration signal C1, and the respiration signal C2 based on continuity of a waveform and correlation of signal segments, to obtain a 180-second respiration signal of the user 1 shown in (a) in FIG. 10 and a 180-second respiration signal of the user 2 shown in (b) in FIG. 10.

Step 405a: The routing device determines sleep monitoring results of a plurality of users based on long-cycle respiration signals of the plurality of users.

During specific implementation, refer to step 405. To be specific, a sleep monitoring result of each user is determined based on the long-cycle respiration signal of the user.

Optionally, the sleep monitoring results of the plurality of users may include a room-level sleep monitoring result. The room-level sleep monitoring result may include a room-level state, and the room-level state may be classified into a room-level sleep state or a room-level non-sleep state.

If all users in a room are in a fall-asleep stage, the state is the room-level sleep state. If at least one user is not in a fall-asleep stage, for example, is in a get-up stage or is in a nocturnal wakefulness state, the state is the room-level non-sleep state.

The sleep monitoring results of the plurality of users may further include room-level sleep duration, and the room-level sleep duration may be duration from a time at which a last user in the room enters the fall-asleep stage to a time at which one user is in the get-up stage.

The sleep monitoring results of the plurality of users may further include a fall-asleep time point by each of the plurality of users, a stay-awake time point of each of the plurality of users, a respiration rate and respiration stability of each of the plurality of users, and the like.

In the foregoing embodiments provided in this application, the method provided in the embodiments of this application is described from a perspective of the terminal serving as an execution body. To implement functions in the method provided in the foregoing embodiments of this application, the terminal may include a hardware structure and/or a software module, to implement the functions in a form of the hardware structure, the software module, or a combination of the hardware structure and the software module. Whether a function in the foregoing functions is performed in a form of the hardware structure, the software module, or the combination of the hardware structure and the software module depends on particular applications and design constraints of the technical solutions.

Based on the foregoing embodiments and a same concept, an embodiment of this application further provides a routing device, configured to perform the steps performed by the routing device in the foregoing method embodiments. For related features, refer to the foregoing method embodiments. Details are not described herein again.

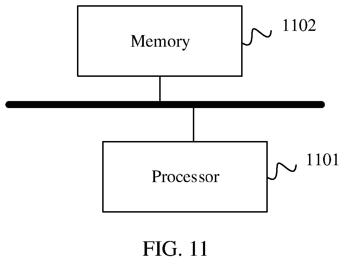

Refer to FIG. 11. The routing device includes one or more processors 1101 and one or more memories 1102. The memory 1102 stores program instructions. When the program instructions are executed by the device, steps of the method in embodiments of this application may be implemented.

The processor 1101 may be a general-purpose processor, a digital signal processor (digital signal processor, DSP), an application-specific integrated circuit (application-specific integrated circuit, ASIC), a field programmable gate array (field programmable gate array, FPGA), or another programmable logic device, a discrete gate or transistor logic device, or a discrete hardware component. The processor may implement or perform the methods, steps, and logical block diagrams that are disclosed in embodiments of this application. The general-purpose processor may be a microprocessor, or the processor may be any conventional processor or the like. The steps of the method disclosed with reference to embodiments of this application may be directly performed by a hardware decoding processor, or may be performed by using a combination of hardware and software modules in a decoding processor. The software module may be located in a mature storage medium in the art, for example, a random access memory (random access memory, RAM), a flash memory, a read-only memory (read-only memory, ROM), a programmable read-only memory, an electrically erasable programmable memory, or a register. The storage medium is located in the memory, and the processor reads the instructions in the memory and completes the steps of the foregoing method in combination with hardware of the processor.

For related features of a specific implementation of the apparatus, refer to the foregoing method part. Details are not described herein again.

Based on a same technical concept, an embodiment of this application further provides a chip. The chip is coupled to a memory in a device, so that the chip, when running, invokes program instructions stored in the memory, to implement the foregoing method in embodiments of this application.

Based on a same technical concept, an embodiment of this application further provides a computer storage medium. The computer-readable storage medium includes a computer program. When the computer program is run on an electronic device, the electronic device is enabled to perform the foregoing method in embodiments of this application.

Based on a same technical concept, an embodiment of this application further provides a computer program product. The computer program product includes instructions. When the instructions are executed, a computer is enabled to perform the foregoing method in embodiments of this application.

Various embodiments of this application may be used separately, or may be used in combination, to achieve different technical effects.

As described above, the foregoing embodiments are merely used to describe the technical solutions of this application in detail. However, descriptions in the foregoing embodiments are merely used to help understand the method in embodiments of this application, and should not be construed as a limitation on embodiments of this application. Any variation or replacement readily figured out by persons skilled in the art shall fall within the protection scope of embodiments of this application.

According to the context, the term "when" used in the foregoing embodiments may be interpreted as a meaning of "if", "after", "in response to determining", or "in response to detecting". Similarly, according to the context, the phrase "when it is determined that" or "if (a stated condition or event) is detected" may be interpreted as a meaning of "if it is determined that", "in response to determining", "when (a stated condition or event) is detected", or "in response to detecting (a stated condition or event)". The terms "include", "contain", "have", and their variants all mean "include but are not limited to", unless otherwise specifically emphasized in another manner.

All or a part of the foregoing embodiments may be implemented by software, hardware, firmware, or any combination thereof. When software is used to implement the embodiments, all or a part of the embodiments may be implemented in a form of a computer program product. The computer program product includes one or more computer instructions. When the computer program instructions are loaded and executed on a computer, all or a part of the procedures or functions according to embodiments of this application are generated. The computer may be a general-purpose computer, a dedicated computer, a computer network, or another programmable apparatus. The computer instructions may be stored in a computer-readable storage medium, or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by the computer, or a data storage device, such as a server or a data center, integrating one or more usable media. The usable medium may be a magnetic medium (for example, a floppy disk, a hard disk, or a magnetic tape), an optical medium (for example, a DVD), a semiconductor medium (for example, a solid-state drive), or the like.

It should be noted that a part of this patent application document includes content protected by the copyright. The copyright owner reserves the copyright except for making copies of the patent documents of the Patent Office or content of the patent documents recorded by the Patent Office.

## Claims

1. A sleep monitoring method, comprising:
receiving, by a routing device, wireless signal flows from at least two smart devices in a space in which a target user is located, wherein the wireless signal flow comprises channel state information for representing a sleep feature of the target user;
determining, by the routing device, a target device from the at least two smart devices based on the wireless signal flows of the at least two smart devices; and
determining, by the routing device, a sleep monitoring result of the target user based on a wireless signal flow of the target device.

2. The method according to claim 1, wherein the determining a target device from the at least two smart devices comprises:
determining, from the at least two smart devices as target devices, top N smart devices whose respiration detection rates are greater than or equal to a first threshold and whose respiration detection rates are highest, wherein N is a positive integer;
determining N smart devices closest to the target user from the at least two smart devices as target devices; or
randomly selecting N smart devices from the at least two smart devices as target devices.

3. The method according to claim 1, wherein the wireless signal flow of the target device carries Wi-Fi CSI information; and the determining, by the routing device, a sleep monitoring result of the target user based on a wireless signal flow of the target device comprises:
determining, by the routing device, body motion feature information and respiration feature information of the target user based on the Wi-Fi CSI information; and
determining the sleep monitoring result of the target user based on the body motion feature information and the respiration feature information, wherein the sleep monitoring result comprises each sleep stage of the user and a sleep quality assessment result.

4. The method according to any one of claims 1 to 3, wherein the method further comprises:
if a respiration detection rate of the target device is less than the first threshold within preset duration, sending, by the routing device, first prompt information to a terminal device, wherein the first prompt information is used to prompt the user to adjust the target device.

5. The method according to claim 4, wherein the method further comprises:
monitoring, by the routing device, a respiration detection rate of another smart device other than the target device in the at least two smart devices; and
if a smart device whose respiration detection rate is greater than the first threshold exists in the another smart device, sending, by the routing device, second prompt information to the terminal device, wherein the second prompt information is used to prompt the user to replace the target device.

6. The method according to any one of claims 1 to 5, wherein the method further comprises:
sending, by the routing device, the sleep monitoring result of the target user to the terminal device.

7. The method according to any one of claims 1 to 6, wherein the space in which the target user is located further comprises at least one other user; and the determining, by the routing device, a sleep monitoring result of the target user based on a wireless signal flow of the target device comprises:
separating, by the routing device, a respiration signal of the target user and a respiration signal of the at least one other user from the wireless signal flow;
determining, by the routing device, the sleep monitoring result of the target user based on the respiration signal of the target user; and
determining, by the routing device, a sleep monitoring result of each of the at least one other user based on a respiration signal of each of the at least one other user.

8. The method according to claim 7, wherein the method further comprises:
sending, by the routing device, a room-level sleep monitoring result to the terminal device, wherein the room-level sleep monitoring result comprises a room-level sleep state, room-level sleep duration, and fall-asleep time points, respiration rates, and respiration stability of the target user and each of the at least one other user.

9. An electronic device, comprising:
a processor, a memory, and one or more programs, wherein
the one or more programs are stored in the memory, the one or more programs comprise instructions, and when the instructions are executed by the processor, the electronic device is enabled to perform steps of the method according to any one of claims 1 to 8.

10. A computer-readable storage medium, wherein the computer-readable storage medium is configured to store a computer program, and when the computer program is run on a computer, the computer is enabled to perform the method according to any one of claims 1 to 8.

11. A computer program product, comprising a computer program, wherein when the computer program is run on a computer, the computer is enabled to perform the method according to any one of claims 1 to 8.
